# EUROPEAN PATENT APPLICATION

(11) **EP 3 858 978 A1**
(43) Date of publication of application: **04.08.2021**
(21) Application number: 19867147.1
(22) Date of filing: 27.09.2019
(51) Int. Cl.: C12N 1/38, A23C 9/123, A23L 5/00, B21B 3/02, C12N 1/00, C12N 11/14, C12Q 1/02, C21D 9/46

(54) **METALLIC MATERIAL, METHOD FOR CONTROLLING RESPONSE OF FERMENTING MICROORGANISM AND METHOD FOR MANUFACTURING FERMENTED FOOD**

(30) Priority: 28.09.2018 JP 2018183688
(71) Applicant: Komatsuseiki Kosakusho Co., Ltd., Suwa-City Nagano Prefecture, 392-0012 (JP)
(72) Inventor: KOMATSU, Takafumi, Suwa-shi, Nagano 392-0012 (JP); YUSA, Fumie, Suwa-shi, Nagano 392-0012 (JP)
(74) Representative: HGF
(86) International application number: PCT/JP2019/038069
(87) International publication number: WO 2020/067392

(57) **Abstract**

Provided is a metal material capable of optimizing a response of a fermentative microorganism, a method of controlling a response of a fermentative microorganism, and a method of producing a fermented food product. A metal material includes a crystal grain having an average crystal grain size for controlling a response of a fermentative microorganism. The average crystal grain size of the crystal grain is preferably 100 nm or more and 10 µm or less. The metal material is preferably stainless steel. It is preferable that the response of the fermentative microorganism is adsorption or growth of the fermentative microorganism on the metal material.

## Description

### BACKGROUND OF THE INVENTION

### Field of the Invention

The present invention relates to a metal material, a method of controlling a response of a fermentative microorganism, and a method of producing a fermented food product.

### Description of the Related Art

Metal materials having refined crystal grains are superior in characteristics such as strength, toughness, and corrosion resistance as compared with metal materials having coarse crystal grains. Accordingly, the metal materials are widely used in various industrial applications such as steel plates and medical devices (e.g., Patent Document 1).

### Prior Art Document

### Patent Document

Patent Document 1: JP-A-2005-169454

### SUMMARY OF THE INVENTION

Recently, there is an increasing awareness of health and self-care issues. In view of this, in order to expand the application of metal materials having fine crystal grains, the present inventors have attempted to newly expand the metal materials for food-related applications, particularly for fermented food-related applications. The metal materials can be expected to be used as bioreactors, constituent parts of the bioreactors, and packaging materials in order to produce fermented food products at a high production rate and enhance the functions of the fermented food products. For this purpose, although it is required to optimize the responses and functions of the fermentative microorganisms, there are wide variety of factors, and thus simple and efficient control of fermentative microorganisms has not been achieved.

An object of the present invention is to provide a metal material capable of optimizing a response of a fermentative microorganism, a method of controlling a response of a fermentative microorganism, and a method of producing a fermented food product.

The present inventors have conducted intensive studies to solve the above problems, and found that there is a difference in affinity between a microorganism and a metal material depending on the type of metal material. As a result of examination based on the finding, they have found that the object can be achieved by adopting the following configuration, and completed the present invention.

In one embodiment, the present invention relates to a metal material including a crystal grain having an average crystal grain size for controlling a response of a fermentative microorganism.

The present inventors have speculated that the grain size of the crystal grain forming the metal material may affect an affinity between the metal material and the fermentative microorganism. Then, they have traced the change in the response of the fermentative microorganism by contacting the fermentative microorganism with the metal material having different average crystal grain sizes. Surprisingly, they have found that when the crystal grain has a specific average crystal grain size, the response of the fermentative microorganism changes significantly. The crystal grain forming the metal material has an average crystal grain size for controlling the response of the fermentative microorganism, so that it is possible to optimize the response of the microorganism in the process of producing a fermented food product using the metal material. As a result, it is possible to improve the production yield of the fermented food product and to enhance the function of the fermented food product.

In one embodiment, the average crystal grain size of the crystal grain is preferably 100 nm or more and 10 µm or less. When such a fine crystal grain is used, the metal material can be applied to a wide range of fermentative microorganisms. Further, the affinity between the metal material and the fermentative microorganism is enhanced, as a result of which it is possible to more efficiently control the response of the fermentative microorganism. In this regard, a method of measuring the average crystal grain size of the crystal grain is as described in examples.

In one embodiment, the metal material is preferably stainless steel in view of easy controllability of the crystal grain size of the crystal grain, versatility, ready availability, processability, and low toxicity.

In one embodiment, it is preferable that the response of the fermentative microorganism is adsorption or growth of the fermentative microorganism on the metal material. The metal material includes a crystal grain having an average crystal grain size suitable for the control of the response of the fermentative microorganism, so that it is possible to promote (i.e., control) the adsorption or growth of the fermentative microorganism as the response of the fermentative microorganism.

In one embodiment, it is preferable that the metal material has an average crystal grain size for giving optimal adsorption or growth of the fermentative microorganism which is determined from a response profile which is a result obtained by cultivating the fermentative microorganism on a metal material having crystal grains with different average crystal grain sizes and plotting a number of the microorganism after the cultivation with respect to the average crystal grain size. According to the metal material, the response of the fermentative microorganism can be optimized just by obtaining a plot of the adsorption number or the growth number of the fermentative microorganisms with respect to different average crystal grain sizes without complicated processes. This can achieve the efficiency improvement of the whole process using the fermentative microorganism.

In one embodiment, the metal material can be suitably used to control the response of at least one selected from the group consisting of lactic acid bacteria, natto bacteria, acetic acid bacteria, koji bacteria, and yeasts, which are typical fermentative microorganisms.

In another embodiment, the present invention relates to a method of controlling a response of a fermentative microorganism using a metal material including a crystal grain, the method including:
providing a plurality of the metal materials whose crystal grains have different average crystal grain sizes to each other;
contacting the plurality of metal materials with the fermentative microorganism;
obtaining a response profile of the fermentative microorganism for each of the average crystal grain sizes of the crystal grains of the plurality of metal materials after the contact; and
determining the average crystal grain size of the crystal grain for giving an optimal response of the fermentative microorganism based on the response profile.

The method of controlling a response of a fermentative microorganism is based on a novel phenomenon that the response of the fermentative microorganism changes depending on the average crystal grain size of the crystal grain forming the metal material, and specificity (extreme value) occurs in the response profile of the fermentative microorganism with respect to the average crystal grain size. In the method of controlling a response of a fermentative microorganism, the average crystal grain size of the crystal grain for giving the optimal response of the fermentative microorganism can be easily determined by reading the extreme value in the response profile obtained by plotting the response of the fermentative microorganism for each of the different average crystal grain sizes. Consequently, it is possible to efficiently and easily achieve the optimization of the response of the fermentative microorganism.

In another embodiment, the control of the response of the fermentative microorganism may be adsorption or growth of the fermentative microorganism on the metal material. In this case, the response profile is preferably a plot of a number of the fermentative microorganism after the contact with respect to the average crystal grain size of the crystal grain. Further preferably, the optimal response is maximization of the number of the fermentative microorganism. In this manner, the extreme value (the local maximum value in this case) of the response profile is read so that it is possible to numerically maximize the control of the response of the fermentative microorganism and to easily and stably provide the necessary number of the fermentative microorganism suitable for the fermentation process.

In another embodiment, the method of controlling a response of a fermentative microorganism can be suitably applied to at least one selected from the group consisting of lactic acid bacteria, natto bacteria, acetic acid bacteria, koji bacteria, and yeasts, which are typical fermentative microorganisms.

In another embodiment, the average crystal grain size of the crystal grain is preferably 100 nm or more and 10 µm or less. When such a fine crystal grain is used, the metal material can be applied to a wide range of fermentative microorganisms. Further, the affinity between the metal material and the fermentative microorganism is enhanced, as a result of which it is possible to more efficiently control the response of the fermentative microorganism.

In another embodiment, the metal material is preferably stainless steel. This is because stainless steel is excellent in easy controllability of the crystal grain size of the crystal grain, versatility, ready availability, processability, and low toxicity.

In still another embodiment, the present invention relates to a method of producing a fermented food product including contacting a fermentative microorganism with the metal material.

In the method of producing a fermented food product, the metal material capable of controlling the response of the fermentative microorganism is contacted with the fermentative microorganism to optimize the response of the fermentative microorganism. Consequently, it is possible to promote the production efficiency of the fermented food product and enhance the function of the fermented food product.

### BRIEF DESCRIPTION OF THE DRAWINGS

Fig. 1A is an example of a response profile obtained by plotting a response amount of a fermentative microorganism with respect to an average crystal grain size of a crystal grain.
Fig. 1B is another example of the response profile obtained by plotting the response amount of the fermentative microorganism with respect to the average crystal grain size of the crystal grain.
Fig. 1C is still another example of the response profile obtained by plotting the response amount of the fermentative microorganism with respect to the average crystal grain size of the crystal grain.
Fig. 2 is a response profile obtained by plotting the number of lactic acid bacteria after cultivation with respect to the average crystal grain size of a plate-shaped metal material in Example 1.
Fig. 3 is a response profile obtained by plotting the number of yeasts after cultivation with respect to the average crystal grain size of a plate-shaped metal material in Example 2.
Fig. 4 is a response profile obtained by plotting the number of lactic acid bacteria after cultivation with respect to the average crystal grain size of a line-shaped metal material in Example 3.

### DETAILED DESCRIPTION OF THE PREFERRED EMBODIMENTS

The metal material, the method of controlling a response of a fermentative microorganism, and the method of producing a fermented food product according to one embodiment of the present invention will be described hereinbelow. The present invention is not limited to these embodiments.

### <<Metal Material>>

The crystal grain forming the metal material according to this embodiment has an average crystal grain size for controlling the response of the fermentative microorganism. The present invention is based on a novel concept utilizing the phenomenon that the response of the fermentative microorganism in contact with the metal material changes significantly when the crystal grains have a specific average crystal grain size. The response of the fermentative microorganism refers to any reaction that a target fermentative microorganism exhibits in the process utilizing the fermentative microorganism. Examples of responses include the growth and immobilization (adsorption), aggregation (agglutination), dispersion (disaggregation) of the fermentative microorganism, death of the fermentation microorganism according to the circumstances, the productions of fermentation products (e.g., alcohol, lactic acids, amino acids) and functional ingredients (e.g., proteins, lipids, peptides) from fermentative microorganisms, and the expression of genes from fermentative microorganisms. According to the metal material of this embodiment, it is possible to optimize the response of microorganism in the process of producing a fermented food product using the metal material. As a result, it is possible to improve the production yield of the fermented food product and to achieve the enhancement of the function.

The average crystal grain size for controlling the response of the fermentative microorganism can be determined by the method of controlling a response of a fermentative microorganism to be described later.

Known metal materials for food-related applications may be used, and examples of the metal materials include iron, stainless steel, aluminum, silver, copper, titanium, tin, nickel, zinc, chromium, and alloys of these metal materials. Among them, stainless steel is preferable in view of easy controllability of the crystal grain size of the crystal grain, versatility, ready availability, processability, and low toxicity. The stainless steel is not particularly limited, and may be any of martensitic stainless steel, ferritic stainless steel, austenitic stainless steel, austenite/ferrite stainless steel, and precipitation hardening stainless steel.

The average crystal grain size of the crystal grain is preferably 100 nm or more and 10 µm or less, more preferably 200 nm or more and 5 µm or less, and still more preferably 500 nm or more and 2 µm or less. The metal material is made of such a fine crystal grain, which allows for the application to a wide range of fermentative microorganisms. Further, it is possible to enhance the affinity between the metal material and the fermentative microorganism and to more efficiently control the response.

As the method of adjusting the average crystal grain size of the crystal grain, a known refinement method can be adopted. Examples of the method include a rolling process for the metal raw material before refinement, a shearing process, a compression process, a deforming process, and a combination of the processes. In this case, cooling or heating may be carried out, or refinement may be carried out in an atmosphere in the presence or absence of a specific gas (such as oxygen or nitrogen). Generally, the refinement is progressed by heating leading to plastic deformation and recrystallization by cooling. The above procedure is carried out once or repeated multiple times, thereby obtaining a desired average crystal grain size.

The shape of the metal material is not particularly limited, and any shape such as a plate shape, a line shape, a rod shape, a spherical shape or a cylindrical shape can be adopted.

The fermentative microorganism is not particularly limited as long as it is a microorganism used for fermented food products. Fermentative microorganisms having a favorable effect on food and fermentative microorganisms which produce a factor having a beneficial effect on the human body are preferable. Particularly, the fermentative microorganism is preferably at least one selected from the group consisting of lactic acid bacteria, natto bacteria, acetic acid bacteria, koji bacteria, and yeasts.

### <<Method of Controlling Response of Fermentative microorganism>>

The method of controlling a response of a fermentative microorganism according to this embodiment is a method which controls a response of a fermentative microorganism using the metal material including the crystal grain. The method includes a metal material providing step, a contacting step, a response profile obtaining step, and an average crystal grain size determining step. Each of the steps will be described in order below.

### (Metal Material Providing Step)

In the step, a plurality of metal materials whose crystal grains have different average crystal grain sizes to each other are provided. For example, a plurality of stainless steel pieces are provided in which each average crystal grain size of crystal grains is adjusted within a range of 100 nm to 10 µm. The number of the average crystal grain size is preferably large in that it is easy to read the extreme value of the profile of the response of the fermenting microorganism. In the case of a plate-shaped metal material, typical average crystal grain size values are as follows: 0.5 µm (500 nm); 1 µm; 1.5 µm; 2 µm; 3 µm; and 9 µm. In the case of a line-shaped (wire-shaped) metal material, typical average crystal grain size values are as follows: values selected from a ranges of 0.5 µm to 0.8 µm, 1 µm to 2 µm, 2 µm to 3 µm, 4 µm to 6 µm, and 8 µm to 10 µm.

### (Contacting Step)

In the step, each of the plurality of metal materials is contacted with the fermentative microorganism. The mode of contact is not particularly limited. The fermentative microorganism may be directly placed on the surface of the metal material, a dispersion liquid (or suspension) of fermentative microorganism may be placed on the surface of the metal material, the metal material may be introduced into the dispersion liquid (or suspension) of fermentative microorganism, and the fermentative microorganism (including the dispersion liquid of fermentative microorganism) may be placed in a container formed of the metal material. Treatments such as heating, cooling, stirring, and shaking may be carried out in combination therewith, if necessary. The mode of contact can be appropriately selected according to the response of the target fermentative microorganism.

Hereinafter, the mode of contact using a lactic acid bacterium as the fermentative microorganism will be described. Regarding other fermentative microorganisms, various conditions may be set using common technical knowledge.

The contact time may be the time that the response of the target fermentative microorganism (lactic acid bacterium in this case; the same applies hereafter.) can be sufficiently obtained. A long period (such as weeks or months) and a short period (such as seconds or minutes) may be used as the contact time required to obtain the response of the fermentative microorganism. For example, in the case of inoculum cultivation or inoculation, the normal contact time is usually 1 second or more and 24 hours or less, preferably 1 minute or more and 20 hours or less, and more preferably 1 hour or more and 18 hours or less. For example, in the case of food production (fermentation), the contact time is usually 12 hours or more and 6 weeks or less, preferably 1 day or more and 4 weeks or less, and more preferably 3 days or more and 3 weeks or less.

For example, in the case of inoculum cultivation and food production (fermentation), the temperature condition at the time of contact may be preferably 20°C or more and 50°C or less.

Other conditions at the time of contact are not limited. When the fermentative microorganism is an anaerobic bacterium, it is preferable that, in a non-aerated state, the anaerobic bacterium is placed or weakly stirred so as to contact with the metal material. In order to keep the components other than the microorganism and the pH constant, cultivation may also be carried out while contacting the anaerobic bacterium with the metal material by weak stirring (stirring to the extent that the medium does not foam).

When the fermentative microorganism is an aerobic bacterium, it is preferable that, in an air atmosphere, the aerobic bacterium is placed or weakly stirred so as to contact with the metal material. In order to keep the components other than the microorganism and the pH constant, cultivation may also be carried out while contacting the aerobic bacterium with the metal material by weak stirring (stirring to the extent that the medium does not foam). The amount of dissolved oxygen at the time of contact can be appropriately adjusted according to the bacteria.

The pH of the environment at the time of contact may be controlled to be in a range of from 4 to 8, preferably from 4.3 to 7, and more preferably from 4.5 to 6.

The contacting step can be carried out in a medium containing carbon sources (such as glucose and fructose), nitrogen sources (such as polypeptone, yeast extract, and malt extract), vitamins, inorganic salts or food materials (e.g., milk constituents) containing nutrients. More specifically, it is possible to use the following nutritional sources.

### (1) Carbon Source

Examples of carbon sources include monosaccharides such as glucose and fructose; disaccharides such as lactose, sucrose and maltose; oligosaccharides; polysaccharides such as cellulose, amylose, chitin, agarose; organic acids such as acetic acid and propionic acid; and alcohols such as ethanol and propanol. Among them, glucose, fructose, lactose, sucrose and maltose are preferable, and glucose, lactose and maltose are more preferable.

The concentration of the carbon source at the time of contact is not particularly limited. The concentration is, for example, in a range of from 0.5 to 20 (w/v)%, preferably from 2 to 15 (w/v)%, and more preferably from 5 to 13 (w/v)%.

### (2) Nitrogen Source

### (i) Peptone

The term "peptone" refers to a substance including a plurality of amino acids bonded to each other, which is obtained by hydrolysis, enzymatic decomposition or fermentation of proteins. There are peptones derived from various species, and examples of peptones include meat extracts, casein peptone, fish peptone, soy peptone, pea peptone, wheat peptone, barley peptone, cottonseed peptone. Among them, casein peptone and soy peptone are preferably used in the present invention.

The concentration of peptone in the medium is not particularly limited. The concentration may be in a range of from 0.5 to 10 (w/v)%, preferably from 0.8 to 8 (w/v)%, and more preferably from 1 to 5 (w/v)%.

### (ii) Yeast Extract

A yeast extract is one obtained by extracting an active ingredient in yeast by self-digestion, enzyme treatment, hot water treatment or the like. Examples of yeast extracts include extracts derived from baker's yeast, beer yeast, and torula yeast. Any of the yeasts may be used in the present invention.

The concentration of the yeast extract in the medium is not particularly limited. The concentration may be in a range of from 0.2 to 10 (w/v)%, preferably from 0.5 to 8 (w/v)%, and more preferably from 0.8 to 4 (w/v)%.

### (3) Inorganic Salts

The kind of inorganic salts that may be present at the time of contact is not particularly limited. Examples of inorganic salts include salts of acetic acid, phosphoric acid, sulfuric acid, carbonic acid, and chloride of potassium, magnesium, sodium, calcium, manganese, cobalt, nickel, molybdenum, tungsten, iron, zinc, copper. Particularly preferable is at least one selected from the group consisting of potassium chloride, potassium dihydrogen phosphate, dipotassium hydrogen phosphate, magnesium sulfate, magnesium phosphate, sodium chloride, sodium acetate, calcium chloride, calcium carbonate, manganese sulfate, ferrous sulfate, zinc sulfate, and copper sulfate.

The amount of the inorganic salts to be added is not limited and can be appropriately selected by those skilled in the art. For example, in the case of using a plurality of inorganic salts, the total amount of the inorganic salts is from 0.1 to 1.6 (w/v)%, preferably from 0.3 to 1.3 (w/v)%, and more preferably from 0.5 to 1 (w/v)%.

### (4) Others

In addition, vitamins and antifoaming agents for preventing foaming at the time of contact may be added.

### (Response Profile Obtaining Step)

In this step, after contacting the metal material with the fermentative microorganism, the response profile of the fermentative microorganism is obtained for each of the average crystal grain sizes of the crystal grains of the metal material. The response profile of the fermentative microorganism can be obtained by taking the average crystal grain size of the crystal grain on the x axis and taking items corresponding to the kind of the response amount of the fermentative microorganism on the y axis to create a two-dimensional plot. Further, a three-dimensional plot may be created by taking the average crystal grain size of the crystal grain on the x-axis, taking other factors (e.g., temperature) that affect the response amount on the y axis, and the items corresponding to the kind of the response amount of the fermentative microorganism on the z axis. Examples of items corresponding to the response amount of the fermentative microorganism include the number of growth of the fermentative microorganism, the number of immobilization (number of adsorption) of the fermentative microorganism on the metal material, the aggregation degree (agglutination degree), the dispersion degree (disaggregation degree), and the production amount of functional ingredients (e.g., proteins) and expression level of genes from fermentative microorganisms as described above.

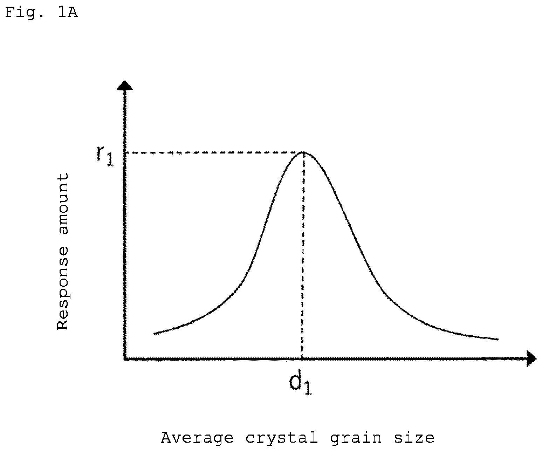

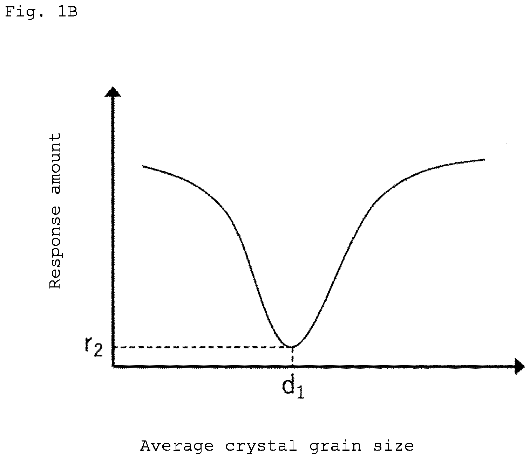

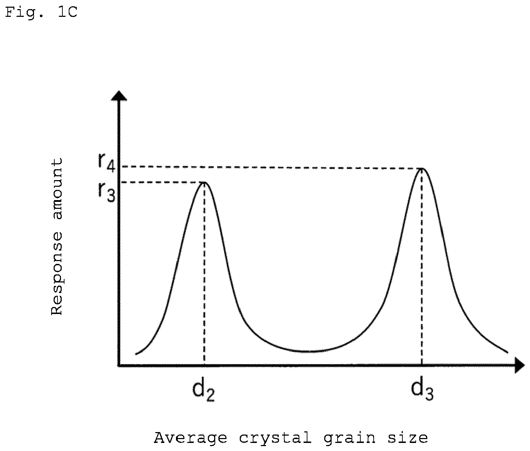

Figs. 1A, 1B, and 1C each show an example of the response profile obtained by plotting the response amount of the fermentative microorganism with respect to the average crystal grain size of the crystal grain. In Fig. 1A, it can be seen that when the average crystal grain size is d₁, the response amount of the fermentative microorganism takes a local maximum value (the maximum value in this case) r₁. In Fig. 1B, contrary to Fig. 1A, it can be seen that when the average crystal grain size is d₁, the response amount of the fermentative microorganism takes a local minimum value (the minimum value in this case) r₂. In Fig. 1C, it can be seen that when the average crystal grain sizes are d₂ and d₃, the response amount of the fermentative microorganism takes two local maximum values r₃ and r₄, respectively (the maximum value is r₄ in this case). In this regard, each of the figures shows a scatter diagram connecting the response amounts with smooth lines, however, the present invention is not limited to this. Further, the response amounts can be displayed by a bar graph or any other arbitrary method.

### (Average Crystal Grain Diameter Determining Step)

In the step, the average crystal grain size of the crystal grain for giving the optimal response of the fermentative microorganism is determined based on the response profile obtained in the above procedure. Whether or not the response is the optimal response may be judged according to the type of response. This determination procedure will be described with reference to Figs. 1A, 1B, and 1C.

In a case in which the response of the fermentative microorganism is, for example, the growth of the fermentative microorganism, the immobilization (adsorption) of the fermentative microorganism on the metal material, or the production of functional ingredients, it is generally preferable that the response amount is increased. Therefore, the average crystal grain size of the crystal grain for giving the optimal response of the fermentative microorganism can be obtained by reading the average crystal grain size for giving the maximum value from the response profiles of Figs. 1A and 1C. In other words, in Fig. 1A, it can be said that the average crystal grain size optimal for the growth and immobilization of the fermentative microorganism and the production of functional ingredients is d₁. In Fig. 1C, the average crystal grain size d₃ which gives the larger response amount r₄ of the two local maximum values may be read, and the read size may be defined as an average crystal grain size optimal for the growth of the fermentative microorganism. Alternatively, when it is possible to judge that the two local maximum values (response amounts r₃ and r₄) satisfy the predetermined criteria, both the average crystal grain sizes d₂ and d₃ can be defined as the average crystal grain sizes optimal for the growth of the fermentative microorganism.

In this manner, the extreme value (the local maximum value in this case) of the response profile is read so that it is possible to numerically or quantitatively maximize the control of the response of the fermentative microorganism and to easily and stably provide the necessary number of the fermentative microorganism suitable for the fermentation process.

In a case in which the response of the fermentative microorganism is, for example, the agglutination of the fermentative microorganism, it is generally preferable that the response amount is reduced or suppressed. Therefore, the average crystal grain size of the crystal grain for giving the optimal response of the fermentative microorganism can be obtained by reading the average crystal grain size for giving the local minimum value from the response profile of Fig. 1B. In other words, in Fig. 1B, it can be said that the optimal average crystal grain size for suppressing the aggregation of fermentative microorganism is d₁.

### <<Method of Producing Fermented Food Product>>

The method of producing a fermented food product according to this embodiment includes contacting a fermentative microorganism with the metal material. The fermented food product is not particularly limited, and examples of fermented food products include the whole range of foods obtained through a fermentation process of fermenting raw materials by the action of fermentative microorganisms. Specific examples of fermented food products include yogurt, lactic acid bacteria beverage, cheese, bread, natto, soy sauce, miso, pickles, dried bonito, black tea, kimchi, sake, and fermented vinegar.

Generally, the method of producing a fermented food product includes the steps of: providing a raw material (providing step); contacting the raw material with a fermentative microorganism to prepare a raw material for fermentation (preparing step); and fermenting the raw material for fermentation (fermenting step). Furthermore, the method of producing a fermented food product according to this embodiment includes a step of cultivating the fermentative microorganism (cultivating step). Although the step of contacting the metal material with the fermentative microorganism may be provided in any step of the fermentation process, the step is preferably at least one of the cultivating step and the fermenting step. In the step, the metal material is contacted with the fermentative microorganism to initiate the optimal response of the fermentative microorganism so that it is possible to promote the production efficiency of the fermented food product and enhance the function of the fermented food product.

As a specific example, a method of producing yogurt will be described hereinbelow. First, a dairy ingredient is provided as a raw material. The dairy ingredient is not particularly limited as long as it is a material made from milk as a basic ingredient. In addition to milk (such as bovine milk and goat milk), skimmed milk powder, condensed milk, fresh cream, milk protein, and the like may also be used. Carbohydrates, stabilizers, emulsifiers, acidulants, pH adjusters, flavoring agents, coloring agents, flavor adjusters, antioxidants, and the like may be added to the dairy ingredients, if necessary.

Then, the provided dairy ingredients are contacted with lactic acid bacteria to prepare a raw material for fermentation. Lactic acid bacteria (also referred to as starters) are not particularly limited, and lactic acid bacteria commonly used for the production of yogurt may be used. Examples of lactic acid bacteria which can be used include the genus Lactobacillus such as Lactobacillus casei, Lactobacillus acidophilus, Lactobacillus gasseri, Lactobacillus zeae, Lactobacillus johnsonii, Lactobacillus delbrueckii, Lactobacillus bulgaricus; the genus Streptococcus such as Streptococcus thermophilus, Streptococcus cremoris, Streptococcus lactis, and Streptococcus diacetylactis; the genus Lactococcus such as Lactococcus lactis, Lactococcus plantarum, and Lactococcus raffinolactis; the genus Leuconostoc such as Leuconostoc mesenteroides, Leuconostoc lactis, and Leuconostoc cremoris; the genus Enterococcus such as Enterococcus faecalis and Enterococcus faecium; and the genus Pediococcus such as Pediococcus cremoris. The lactic acid bacteria may be used singly, or in combination of two or more kinds thereof.

Subsequently, the raw material for fermentation is filled into a container to perform fermentation. Fermentation is performed at 25 to 45°C for 2 to 72 hours, but the condition varies depending on raw materials and lactic acid bacteria. In this embodiment, the above-described metal materials may be suitably used as the material of the container.

In addition to the process, an inoculum of lactic acid bacteria may be cultivated.

### <<Other Embodiments>>

Other embodiments are illustrated below.
[1] A method of producing a fermented food product, using a metal material including a crystal grain,
   the method comprising:
   providing a plurality of the metal materials having a crystal grains with different average crystal grain sizes;
   contacting the plurality of metal materials with a fermenting microorganism;
   obtaining a response profile of the fermenting microorganism for each of the average crystal grain sizes of the crystal grains of the plurality of metal materials after the contact;
   determining the average crystal grain size of the crystal grain for giving an optimal response of the fermenting microorganism based on the response profile; and
   contacting the metal materials having a determined average crystal grain size of the crystal grain for giving an optimal response of the fermenting microorganism with the fermenting microorganism.
[2] A method of producing a fermented food product, using a metal material including a crystal grain, comprising contacting a metal material having an average crystal grain size of 100 nm or more and 10 µm or less with a fermenting microorganism, wherein a population of the fermenting microorganism is increased by the contact.
[3] The method according to [1] or [2], wherein the fermenting microorganism is at least one selected from the group consisting of lactic acid bacteria, natto bacteria, acetic acid bacteria, koji bacteria, and yeasts.
[4] The method according to [1], wherein the crystal grain has an average crystal grain size of 100 nm or more and 10 µm or less.
[5] The method according to any one of [1] to [4], wherein the metal material is stainless steel.
[6] A method of increasing a yeast population comprising:
   providing a metal material having an average crystal grain size of less than 1 µm; and
   contacting the metal material with a yeast.
[7] The method according to [6], wherein the average crystal grain size is from 0.3 to 0.8 µm inclusive.
[8] A method of increasing a lactic acid bacterium population comprising:
   providing a metal material having an average crystal grain size of 1 µm or more; and
   contacting the metal material with a lactic acid bacterium.
[9] The method according to [8], wherein the average crystal grain size is from 1.2 to 1.8 µm inclusive.

### Examples

Hereinafter, the present invention will be described in detail with reference to examples, but the present invention is not limited to the following examples unless it exceeds the gist of the present invention.

### <Example 1: Adsorption of Lactic Acid Bacteria on (Plate-Shaped) Metal Material>

### <<Providing of Metal Material>>

In order to provide metal materials, stainless steel (SUS 304) was subjected to rolling treatment and thermal recrystallization to adjust the average crystal grain sizes of crystal grains to 0.5 µm, 1 µm, 1.5 µm, 2 µm, 3 µm, and 9 µm, respectively. The metal material had a plate shape having a length of 10 mm, a width of 10 mm, and a thickness of 0.1 mm. The rolling treatment and thermal recrystallization were carried out according to the following procedure. Specifically, the stainless steel (SUS 304) was passed through a rotating mill several times and cold-rolled to about 40 to 65% (compression ratio of about 3 to 15% per time). Then, the resulting stainless steel was subjected to annealing at 600 to 850°C for 10 to 100 seconds (heating rate: 200°C/sec) to recrystallize the stainless steel. Finally, the recrystallized stainless steel was cooled to obtain an austenitic stainless steel (cooling rate: 200 to 400°C/sec.).

### <<Measurement of Average Crystal Grain Size>>

The test sample of the metal material provided above was polished with argon ions using an ion polisher ("IM 4000", manufactured by Hitachi High-Technologies Corporation). Thereafter, the average crystal grain size of the metal material was measured at room temperature in a vacuum environment (1 × 10⁻³ Pa) using an electron microscope ("SU-70", manufactured by Hitachi High-Technologies Corporation) having a crystal orientation analysis function. The size of each crystal grain was determined by determining the area of each crystal grain in an arbitrary measurement range (i.e., the observed image; magnification: 1000 times) and calculating a diameter of a circle, assuming that the shape of the crystal grain is a circle having the same area as the area of the crystal grain. The area of the crystal grain and the diameter of the circle having the same area as the area of the crystal grain were calculated using an image processor ("TSL OMI Analysis 7", manufactured by TSL Solutions). Then, the sum of all crystal grain diameters in the arbitrary measurement range was divided by the number of crystal grains, and the resulting value was defined as an average crystal grain size (nm).

### <<Measurement of Number of Lactic Acid Bacteria Adsorbed on Metal Material>>

As a yogurt material, a commercially available "Meiji Probio Yogurt LG21 (sugar-free)" (containing LG21 Lactobacillus gasseri OLL 2716 (Gram-positive bacillus and facultative anaerobe)) was provided. Yogurt was developed in a petri dish. The metal materials having the average crystal grain sizes were completely embedded in the yoghurt and subjected to an adsorption reaction at 35 ± 2°C for 24 hours. As a control, a polypropylene film (having the same shape as the metal material) was embedded in the yoghurt. After the adsorption reaction, the film was washed directly with 2 to 3 mL of PBS (sterilized) and further washed twice with 15 mL of PBS (sterilized). Then, lactic acid bacteria were liberated in 5 mL of PBS (sterilized) by vortexing for 40 seconds. The undiluted suspension and the 10-fold diluted suspension were developed on 3M Petrifilm Lactic Acid Bacteria Count Plate (LAB Plate), manufactured by 3M Company. A 10⁶-fold diluted yogurt sample and a 10⁷-fold diluted yogurt sample were prepared as controls. Finally, the samples were cultured at 35°C for 46 hours, and the number of the cultured lactic acid bacteria was counted as the number of lactic acid bacteria adsorbed per 1 cm² of the stainless steel piece (CFU/cm² (colony forming unit/cm²).

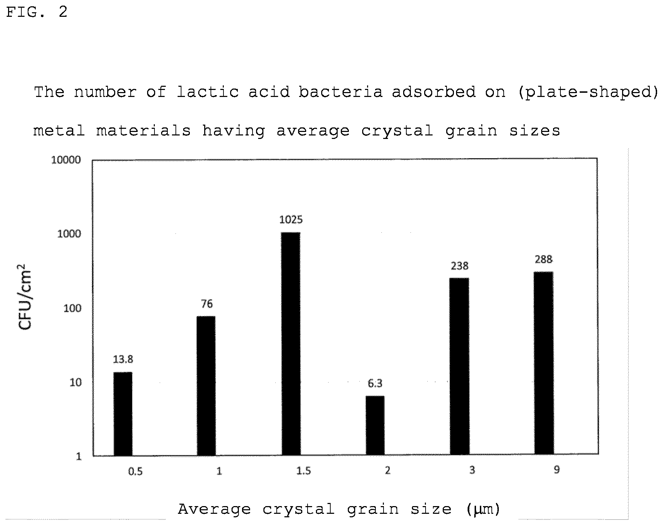

The results are shown in Fig. 2. Fig. 2 shows the response profile obtained by plotting the number of lactic acid bacteria after cultivation with respect to the average crystal grain size in the example. As shown in Fig. 2, the number of the lactic acid bacteria adsorbed on the metal materials was relatively increased when the average crystal grain sizes were 1.5 µm, 3 µm, and 9 µm. In the case of the polypropylene film as the control, the average crystal grain size was 188 CFU/cm². Particularly, the maximum value was obtained when the average crystal grain size was 1.5 µm. It was found that the average crystal grain size optimal for adsorption of lactic acid bacteria was 1.5 µm. The practical application of the results suggests the following possibility; regarding fermentative microorganisms which obtain energy through the fermentation in the absence of oxygen (such as facultative anaerobe), the number of the fermentative microorganisms adsorbed on the metal material is counted, thereby providing an indicator of the degree of the progress of fermentation by the fermentative microorganisms.

### <Example 2: Adsorption of Yeasts on (Plate-Shaped) Metal Material>

### «Providing of Metal Material»

The same metal material as in Example 1 was used.

### <<Measurement of Number of Yeasts Adsorbed on Metal Material>>

One packet (3 g) of commercially available "Dry Yeast, Pioneer-kikaku" for the test was dissolved in 30 mL of (sterilized) PBS equilibrated at 37°C ± 2°C. 3 mL of the resultant solution was uniformly mixed with a skim milk solution (sterilized, equilibrated at 25°C ± 2°C) in which one packet (6 g) of "Skim Milk, Pioneer-kikaku" was dissolved in 300 mL of distilled water, and the reaction solution was provided. The reaction solution was developed in a petri dish. The metal materials having the average crystal grain sizes were completely embedded in the reaction solution and subjected to an adsorption reaction at 35 ± 2°C for 2 hours. As a control, a polypropylene film (having the same shape as the metal material) was embedded in the reaction solution. After the adsorption reaction, the film was washed directly with 2 to 3 mL of PBS (sterilized) and further washed twice with 15 mL of PBS (sterilized). Then, yeasts were liberated in 5 mL of PBS (sterilized) by vortexing for 40 seconds. The undiluted suspension and the 10-fold diluted suspension were developed on 3M Petrifilm Plates For Rapidly Counting Molds and Yeasts (RYM Plate), manufactured by 3M Company. A 10⁴-fold diluted reaction solution and a 10⁵-fold diluted reaction solution were prepared as controls. Finally, the reaction solutions were cultured at 25°C ± 2°C for 48 hours, and the number of the cultured yeasts, adsorbed on 1 cm² of the stainless steel piece (CFU/cm² (colony forming unit/cm²)), was counted.

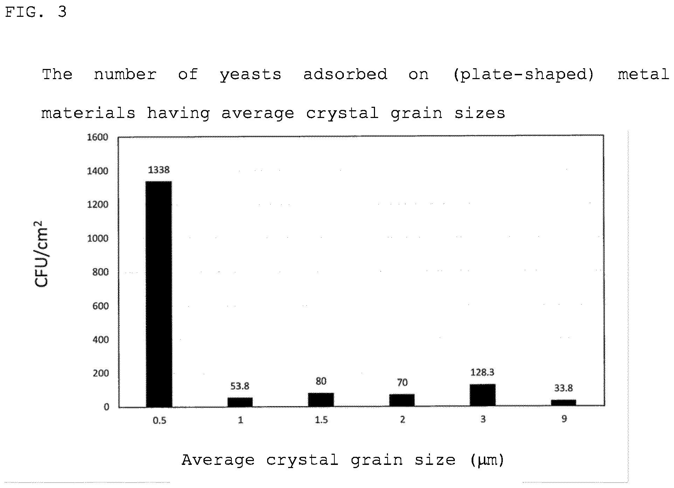

The results are shown in Fig. 3. Fig. 3 shows the response profile obtained by plotting the number of yeasts after cultivation with respect to the average crystal grain size in Example 2. From Fig. 3, it was found that the maximum value of the number of yeasts adsorbed on metal materials was obtained when the average crystal grain size was 0.5 µm, and the average crystal grain size optimal for adsorption of yeasts was 0.5 µm. In the case of the polypropylene film as the control, the number of yeasts was 4725 CFU/cm². The practical application of the results suggest the following possibility; regarding fungi which obtain energy through the fermentation, the number of the fungi adsorbed on the metal material is counted, thereby providing an indicator of the degree of the progress of fermentation by the fungi.

### <Example 3: Adsorption of Lactic Acid Bacteria on (Line-Shaped) Metal Material>

### <<Providing of Metal Material>>

In order to provide metal materials, stainless steel (SUS316L) was subjected to rolling treatment and thermal recrystallization to adjust the average crystal grain sizes of crystal grains to 0.7 µm, 1.5 µm, 2.8 µm, 4.9 µm, and 9.6 µm, respectively. The metal material had a line shape (wire shape) with a diameter of 1 mm and a length of 13 mm. The rolling treatment and thermal recrystallization were carried out according to the following procedure. Specifically, the stainless steel (SUS316L) was passed through a rotating mill several times and cold-rolled to about 40 to 65% (compression ratio of about 3 to 15% per time). Then, the resulting stainless steel was subjected to annealing at 600 to 850°C for 10 to 3600 seconds (heating rate: 200°C/sec) to recrystallize the stainless steel. Finally, the recrystallized stainless steel was cooled to obtain an austenitic stainless steel (cooling rate: 200 to 400°C/sec.). The average crystal grain size was measured by the same procedure as in Example 1.

### <<Measurement of Number of Lactic Acid Bacteria Adsorbed on (Line-Shaped) Metal Material>>

As a yogurt material, a commercially available "Meiji Probio Yogurt LG21 (sugar-free)" (containing LG21 Lactobacillus gasseri OLL 2716 (Gram-positive bacillus and facultative anaerobe)) was provided. Yogurt was developed in a petri dish. The metal materials having the average crystal grain sizes were completely embedded in the yoghurt and subjected to an adsorption reaction at 35 ± 2°C for 2 hours. As a control, a commercially available SUS316L stainless steel (average crystal grain size of 17.4 µm) having the same shape as the metal material was embedded in the reaction solution. After the adsorption reaction, the stainless steel was washed twice with 15 mL of PBS (sterilized) and then lightly rinsed with 1 mL of PBS. Then, lactic acid bacteria were liberated in 5 mL of PBS (sterilized) by vortexing for 40 seconds. The undiluted suspension and the 10-fold diluted suspension were developed on 3M Petrifilm (medium) Plates For Counting Lactic Acid Bacteria (LAB Plate), manufactured by 3M Company. A 10⁶-fold diluted yogurt sample and a 10⁷-fold diluted yogurt sample were prepared as controls. Finally, the samples were cultured at 35°C for 48 hours, and the number of the cultured lactic acid bacteria, adsorbed on 1 mm² of the stainless steel piece (CFU/mm² (colony forming unit/mm²), was counted.

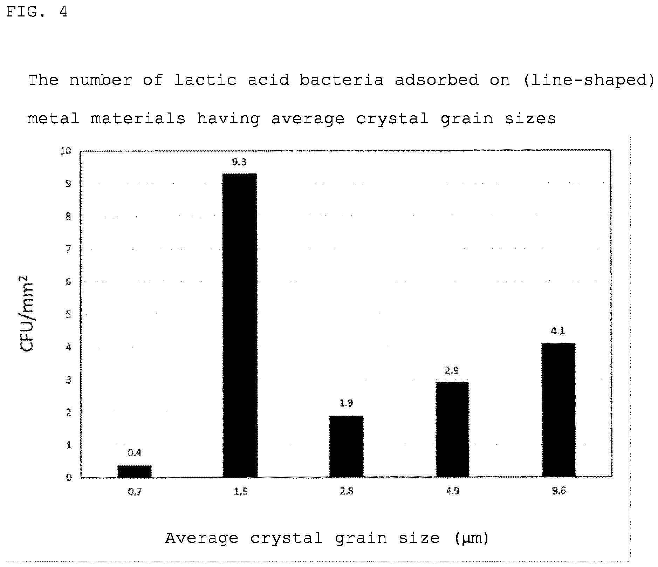

The results are shown in Fig. 4. Fig. 4 shows the response profile obtained by plotting the number of lactic acid bacteria after cultivation with respect to the average crystal grain size in Example 3. As shown in Fig. 4, the number of the lactic acid bacteria adsorbed on the metal materials was relatively increased when the average crystal grain sizes were 1.5 µm, 4.9 µm, and 9.6 µm. The number of the lactic acid bacteria adsorbed on the control was 1.2 CFU/mm². It is presumed that this is partly due to the fact that the average crystal grain size of the commercially available SUS316L material as the control was large, the crystal grain size varied widely, and some parts having lactic acid bacteria adsorbed thereon coexisted with some parts having no lactic acid bacteria adsorbed thereon. Particularly, the maximum value was obtained when the average crystal grain size was 1.5 µm. It was found that the average crystal grain size optimal for adsorption of lactic acid bacteria was 1.5 µm. The practical application of the results suggest the following possibility; regarding fermenting microorganisms which obtain energy through the fermentation in the absence of oxygen (such as facultative anaerobe), the number of the fermenting microorganisms adsorbed on the metal material is counted, thereby providing an indicator of the degree of the progress of fermentation by the fermenting microorganisms.

## Claims

1. A metal material comprising a crystal grain having an average crystal grain size for controlling a response of a fermentative microorganism.

2. The metal material according to claim 1, wherein the crystal grain has an average crystal grain size of 100 nm or more and 10 µm or less.

3. The metal material according to claim 1, wherein the metal material is stainless steel.

4. The metal material according to any one of claims 1 to 3, wherein a response of the fermentative microorganism is an adsorption or growth of the fermentative microorganism on the metal material.

5. The metal material according to any one of claims 1 to 4, wherein the metal material has an average crystal grain size for giving an optimal adsorption or growth of the fermentative microorganism which is determined from a response profile which is a result obtained by cultivating the fermentative microorganism on a metal material having crystal grains with different average crystal grain sizes and plotting a number of the microorganism after the cultivation with respect to the average crystal grain size.

6. The metal material according to any one of claims 1 to 5, wherein the fermentative microorganism is at least one selected from the group consisting of lactic acid bacteria, natto bacteria, acetic acid bacteria, koji bacteria, and yeasts.

7. A method of controlling a response of a fermentative microorganism which controls a response of a fermentative microorganism using a metal material including a crystal grain, the method comprising:
providing a plurality of the metal materials having the crystal grains with different average crystal grain sizes;
contacting the plurality of metal materials with the fermentative microorganism;
obtaining a response profile of the fermentative microorganism for each of the average crystal grain sizes of the crystal grains of the plurality of metal materials after the contact; and
determining the average crystal grain size of the crystal grain for giving an optimal response of the fermentative microorganism based on the response profile.

8. The method according to claim 7, wherein the control of the response of the fermentative microorganism is an adsorption or growth of the fermentative microorganism on the metal material.

9. The method according to claim 8, wherein the response profile is a plot of a number of the fermentative microorganism after the contact with respect to the average crystal grain size of the crystal grain.

10. The method according to claim 9, wherein the optimal response is a maximization of the number of the fermentative microorganism.

11. The method according to any one of claims 7 to 10, wherein the fermentative microorganism is at least one selected from the group consisting of lactic acid bacteria, natto bacteria, acetic acid bacteria, koji bacteria, and yeasts.

12. The method according to any one of claims 7 to 11, wherein the crystal grain has an average crystal grain size of 100 nm or more and 10 µm or less.

13. The method according to any one of claims 7 to 12, wherein the metal material is stainless steel.

14. A method of producing a fermented food product comprising contacting a fermentative microorganism with the metal material according to any one of claims 1 to 6.
